# FASCICULE DE BREVET EUROPEEN

(11) **EP 0 511 092 B1**
(45) Date de publication et mention de la délivrance du brevet: **20.12.1995**
(21) Numéro de dépôt: 92401136.4
(22) Date de dépôt: 22.04.1992
(51) Int. Cl.: A61K 7/48

(54) **Microsphères poreuses enrobées à l'aide d'une huile perfluorée, d'une huile de silicone fluorée ou d'une gomme de silicone et leur utilisation en cosmétique**
Mit perfluoriertem Öl, Silicon-Öl oder Silicon-Gummi überzogene poröse Mikrokugeln und deren kosmetische Anwendung
Porous microspheres enrobed with a perfluorinated oil, a silicon oil or a silicon gum and cosmetic use thereof

(30) Priorité: 22.04.1991 FR 9104932
(43) Date de publication de la demande: 28.10.1992
(73) Titulaire: L'OREAL, 75008 Paris (FR)
(72) Inventeur: Mellul, Myriam, F-94240 L'Hay les Roses (FR); Arnaud, Pascal, F-75009 Paris (FR)
(74) Mandataire: Stalla-Bourdillon, Bernard

(56) Documents cités:
- EP-A- 0 212 870
- EP-A- 0 254 612
- EP-A- 0 340 103
- EP-A- 0 379 409
- EP-A- 0 390 206
- EP-A- 0 407 089
- EP-A- 0 422 984
- WO-A-89/10132
- FR-A- 2 151 170
- FR-A- 2 530 250

## Description

La présente invention a pour objet des microsphères poreuses enrobées d'une huile perfluorée, d'une huile de silicone fluorée ou de gomme de silicone et leur utilisation dans des compositions cosmétiques, en particulier dans des rouges à lèvres, des poudres, des fards à paupières et à joues, des mascaras, des fonds de teint, des crèmes, des lotions et sérums et des déodorants.

Il est bien connu que les huiles perfluorées, en particulier les perfluoropolyéthers apportent des propriétés cosmétiques particulièrement intéressantes mais toutefois leur usage a été considérablement limité du fait de leur insolubilité aussi bien en phase aqueuse que dans les huiles classiques et dans les huiles de silicone.

Pour résoudre ce problème, on a essayé de les incorporer dans des compositions cosmétiques, soit sous forme d'une dispersion, comme par exemple dans des rouges à lèvres, des poudres compactes, des fards à joues, des émulsions huile-dans-l'eau ou eau-dans-l'huile ou encore dans des gels aqueux comme ceci est décrit dans la demande de brevet EP 196 904, soit sous forme d'une émulsion en présence d'un tensioactif, dans du glycérol ou dans une solution aqueuse concentrée d'un polyol de fonctionnalité supérieure à 3 tel que par exemple le sorbitol comme ceci est décrit dans la demande de brevet EP 390206.

A un degré moindre, le problème d'incorporation se pose également pour les silicones fluorées et les gommes de silicone qui sont uniquement solubles dans les huiles de silicone. Les dispersions en phase anhydre ne permettent pas un taux d'incorporation très élevé, dans la mesure où l'affinité des gouttelettes d'huiles perfluorées, de silicones fluorées ou encore de gommes de silicone pour la matrice, sont faibles et peuvent engendrer une fragilisation de la structure de cette matrice comme c'est le cas pour les rouges à lèvres.

D'autre part, l'introduction sous forme émulsionnée ou en dispersion dans un gel aqueux permet d'augmenter la concentration mais nécessite dans le cas d'une émulsion, l'utilisation d'un tensioactif et dans le cas d'une dispersion, une certaine viscosité.

Après d'importantes recherches et de façon surprenante, on a constaté que l'on pouvait résoudre le problème de l'introduction, en plus grande quantité, d'huile perfluorée, de silicone fluorée et de gomme de silicone, sans utiliser de tensioactif, et sans avoir une contrainte en viscosité.

Selon l'invention, la solution consiste à utiliser des microsphères poreuses préalablement enrobées d'une huile perfluorée, d'un silicone fluorée ou d'une gomme de silicone.

De telles microsphères poreuses ainsi enrobées se sont montrées présenter d'excellentes propriétés cosmétiques qui jusqu'à ce jour n'avaient pu être obtenues selon les techniques décrites.

La présente invention a donc pour objet des microsphères poreuses enrobées à l'aide d'une huile perfluorée, d'une huile de silicone fluorée, ou d'une gomme de silicone, l'enrobage étant fixé à la surface externe des dites microsphères.

La substance d'enrobage comme celle-ci sera mieux définie ci-dessous et est de nature telle qu'elle ne pénètre pas l'intérieur des pores des microsphères et ne se fixe qu'à l'extérieur.

Les microsphères poreuses présentent des tailles moyennes de particules inférieures à 800 »m et de préférence comprises entre 0,1 et 300 »m. Ces microsphères sont de forme sphérique ou sphéroïde et possèdent un réseau de pores interconnectes et ouverts sur l'extérieur et éventuellement susceptibles d'être remplis par une phase liquide hydrophile ou lipophile suivant leur nature chimique.

Parmi les microsphères poreuses qui peuvent être utilisées selon l'invention, on peut notamment citer :
- les polyamides tels que la poly-β-alanine décrite dans les brevets FR-83 11609 et FR-87 17573,
- les polyacrylates tels que le "POLYTRAP Q5-6603" vendu par la Société DOW CORNING,
- les polyamides tels que l'"ORGASOL" vendu par la Société ATOCHEM,
- les polyméthacrylates tels que le "MICROPEARL M" vendu par la Société SEPPIC ou "PLASTIC POWDER A" vendu par la Société NIKKOL,
- les copolymères de styrène-divinylbenzène tels que ceux vendus par la Société APS ou "PLASTIC POWDER FP" vendu par la Société NIKKOL,
- les polyéthylènes et polypropylènes tels que l'"ACCUREL EP400" vendu par la Société AKZO,
- les celluloses telles que la "MICROSPHERICAL CELLULOSE" vendue par CHISSO,
- les silicones telles que la "SILICONE POWDER X2-1605" vendue par la Société DOW CORNING,
- les silices telles que le "SILCRON G 640" vendue par la Société SCM, et
- les poudres d'hydroxyapatite telles que le "CERAPOL" vendu par la Société KOKEN.

Selon l'invention, l'enrobage des microsphères poreuses est généralement réalisé en utilisant de 0,1 à 500 % en poids et de préférence de 1 à 300% en poids d'huile perfluorée, d'huile de silicone fluorée, ou de gomme de silicone par rapport au poids des microsphères.

Parmi les huiles perfluorées utilisables selon l'invention, on peut notamment citer celles ayant des viscosités généralement inférieures à 5.10⁻³m²/s à 20°C. Parmi ces huiles, on peut notamment mentionner :
- les perfluoroalcanes de formule générale :

   CₙF₂ₙ₊₂

   avec n > 19
- et les perfluoropolyéthers ayant les formules générales suivantes :
   (a) 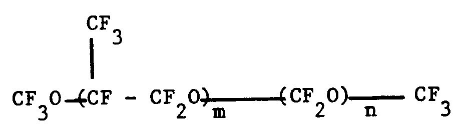 avec le rapport m/n : 5 à 40
   (b) RCF₂O-(C₂F₄O)ₚ-(CF₂O)_{q}-CF₂R
      avec le rapport p/q = 0,5 à 1,5
      R représente un atome de fluor, -COOH, -COOCH₃, -CH₂OH, ou -CH₂O-CH₂-CHOH-CH₂OH et -CH₂(OCH₂CH₂)ₚ-OH avec p = 1 ou 2.
   (c) 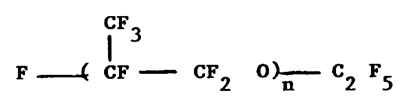 avec n = 10 - 500
   (d) 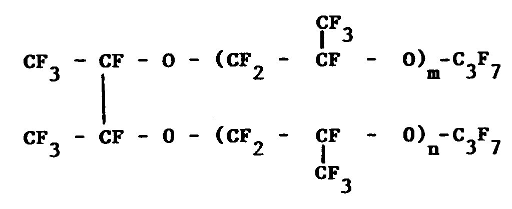 dans laquelle :
      m et n sont des nombres entiers de 0 à 3, tels que le poids moléculaire est supérieur à 1000.

Parmi les huiles de silicones fluorées, on peut notamment citer celles ayant des viscosités comprises entre 1.10⁻⁴ et 1.10⁻² m²/s à 25°C et ayant les formules suivantes :
(a) 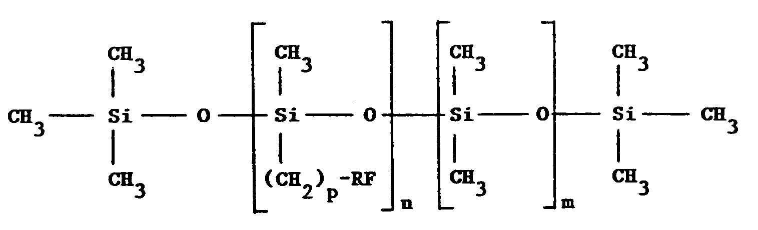 dans laquelle :
   n est un nombre entier de 1 à 300,
   m est un nombre entier de 0 à 150,
   p est un nombre entier de 0 à 5 et
   RF est un radical perfluoroalkyle ayant de 1 à 8 atomes de carbone.
(b) 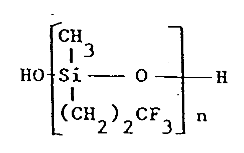 avec n > 3
Enfin, parmi les gommes de silicone, on peut citer celles ayant des viscosités supérieures ou égales à 1.10⁻² m²/s à 25°C et de préférence supérieures ou égales à 5.10⁻² m²/s.

Parmi ces gommes de silicone, on peut notamment citer celles correspondant à la formule générale suivante : 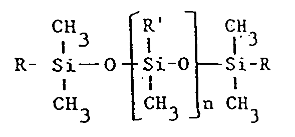
R représente -CH₃,-OH ou -CH=CH₂ et
R' représente -CH₃ ou-C₆H₅
n étant tel que la viscosité est > 1.10⁻² m²/s à 25°C.

Parmi les huiles perfluorées et en particulier parmi les perfluoropolyéthers de formules données ci-dessus, on peut notamment citer les produits vendus par la Société MONTEFLUOS sous les dénominations suivantes : FOMBLIN HC04, FOMBLIN HC25, FOMBLIN HCR, FOMBLIN ZO3, FOMBLIN Z15, FOMBLIN Z25, FOMBLIN Z60, FOMBLIN ZDEAL, FOMBLIN ZDIAC, FOMBLIN ZDOL, FOMBLIN ZTETRAOL et FOMBLIN ZDOL TX, le produit vendu par la Société du PONT de NEMOURS sous la dénomination KRYTOX et le produit vendu par la Société HOECHST sous la dénomination HOSTINERT.

Parmi les huiles de silicone répondant aux formules données ci-dessus, on peut citer les produits vendus par la Société SHINETSU sous les dénominations de X 22819, X22820, X22821 et X22822 ainsi que sous les dénominations de FL100 (4,5.10⁻⁴ m²/s), FL1OO (1.10⁻³ m²/s) et FL100 (1.10⁻² m²/s), les produits vendus par DOW CORNING sous la dénomination de FS 1265 et celui vendu par la Société GENERAL ELECTRIC sous la dénomination FF 150.

Parmi les gommes de silicone, on peut notamment citer la diméthicone vendue par la Société WACKER sous la dénomination de "AK 500000" et le diméthiconol vendu sous la dénomination de "SGM3" par la Société DOW CORNING.

Lorsque les microsphères sont enrobées par des huiles perfluorées ou des huiles de silicones fluorées et que leur formulation nécessite un chauffage en présence d'une phase huileuse comme ceci est le cas pour les rouges à lèvres, les fards à joues ou les émulsions, on utilisera une phase d'enrobage contenant de préférence au moins 10 % en poids d'huiles perfluorées ou d'huiles de silicones fluorées possédant des fonctions polaires telles que :
(a) 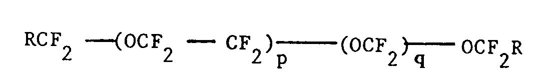 avec le rapport p/q = 0,5 à 1,5 R représente -COOH ou -CH₂O-CH₂-CHOH-CH₂OH
(b) 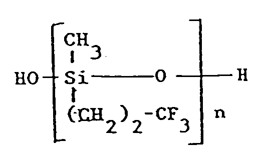 avec n > 3
   afin de renforcer les interactions de la phase d'enrobage avec les microsphères poreuses.

Selon une forme de réalisation préférée de l'invention, les microsphères poreuses préalablement à leur enrobage sont chargées à l'intérieur des pores à l'aide d'une phase hydrophile, lipophile ou perfluorée, celle-ci ne devant pas, bien entendu, être miscible vis-à-vis de la substance d'enrobage et de préférence ne pas l'être non plus vis-à-vis du milieu de dispersion. Selon cette forme de réalisation, on peut donc réaliser des dispersions stables de trois phases non miscibles entre elles sans utiliser de tensioactif.

Il convient par ailleurs de remarquer que la présence d'une phase de chargement permet d'augmenter la quantité de phase d'enrobage.

Selon cette forme de réalisation, la phase hydrophile peut être de l'eau, un alcool polyhydroxylé ou un de leurs mélanges tels que par exemple un mélange d'eau et de glycérol ou de propanediol-1,2 dans des proportions comprises entre 0,1 et 10 fois la masse des microsphères poreuses et de préférence entre 0,1 et 5 fois.

Cette phase hydrophile pourra renfermer des actifs hydrosolubles généralement utilisés dans des compositions cosmétiques ou pharmaceutiques, en particulier des hydratants tels que le D-panthénol, le polyméthacrylate de glycéryle, le hyaluronate de sodium, le sel de sodium de l'acide pyroglutamique, et des vitamines.

Parmi les substances susceptibles de constituer la phase lipophile, on peut notamment citer les huiles classiques, communément utilisées en cosmétique telles que les huiles minérales comme l'huile de paraffine, des huiles végétales telles que l'huile de jojoba ou de ricin, des huiles animales comme le squalane ou encore des huiles de synthèse comme le myristate d'isopropyle, des huiles de silicone ou des huiles fluorées ainsi d'ailleurs que des huiles essentielles ou des extraits de parfum dans des proportions comprises entre 0,1 et 5 fois la masse des microsphères poreuses et de préférence entre 0,1 et 3 fois.

La phase lipophile pourra bien entendu également servir de véhicule à une substance active cosmétique ou pharmaceutique liposoluble en particulier l'acétate d'α-tocophérol, le nicotinate de tocophérol, le β-carotène, le N,N-diéthyltoluamide, les filtres U.V. liposolubles, le ricinoléate de zinc, les antioxydants, les conservateurs, etc.

Quand les microsphères sont chargées d'une phase perfluorée, il s'agit d'une huile perfluorée ayant une masse moléculaire inférieure à 1000 et de préférence inférieure à 500.

Parmi ces huiles perfluorées, on peut citer :
1) celles appartenant au groupe des perfluoroalcanes, des perfluorocycloalcanes, des perfluoropolycycloalcanes et des perfluoro (alkylcycloalcanes).
   Comme exemples de perfluorcalcanes, on peut citer la série des alcanes linéaires tels que le perfluoryclooctane, le perfluorononane ou encore le perfluorodécane.
   Comme exemples de perfluorocycloalcanes et de perfluoro (alcylcycloalcanes), on peut citer la perfluorodécaline, la perfluoro (méthyldécaline), les perfluoro (C₃-C₅-alkylcyclohexanes) tels que le perfluoro (butylcyclohexane), les dérivés du bicyclo [3,3,1] nonane tels que le triméthylbicyclo [3,3,1] nonane, les dérivés perfluorés de l'adamantane tel que le perfluorodiméthyl adamantane et le perfluorotétradécahydrophénanthrène.
2) Celles appartenant au groupe des hydrocarbures perfluorés aromatiques (perfluoroarènes) comme les dérivés perfluorés du naphtalène tels que le perfluoronaphtalène et le perfluorométhyl-1-naphtalène.
3) Celles appartenant au groupe des hydrocarbures perfluorés contenant au moins un hétéroatome, par exemple les amines tertiaires perfluorées comme la perfluorotributylamine, ou des composés hétérocycliques saturés, substitués par des groupements alkyles, comme les perfluoro (alkyl tétrahydropyrannes) tels que la perfluoro (hexyltétrahydropyranne), les perfluoro (alkyltétra-hydrofuranne) tels que le perfluoro (heptyltétrahydrofuranne) et le perfluoro (butyltétrahydrofuranne) ou les dérivés de la morpholine comme la perfluoro (N-pentylmorpholine).
4) Celles appartenant au groupe des perfluoro polyéthers de faible masse moléculaire tels, par exemple, que les prdouits vendus par MONTEFLUOS sous la dénomination GALDEN, en particulier le GALDEN DO2 et le GALDEN DO3.

Selon cette forme de réalisation, l'enrobage des microsphères poreuses chargées est généralement réalisé en utilisant de 0,1 à 500 % en poids et de préférence de 1 à 300 % en poids d'une huile perfluorée, d'une huile de silicone fluorée ou de gomme de silicone par rapport au poids des microsphères, cette quantité variant selon la nature de l'enrobant.

La présente invention a également pour objet divers procédés de préparation des microsphères poreuses enrobées.

Selon un premier procédé, on mélange, dans un récipient adapté, sous agitation, les microsphères poreuses avec la substance d'enrobage à l'état liquide. Ce procédé s'applique essentiellement aux huiles perfluorées et aux huiles de silicones fluorées.

Un autre procédé, utilisé préférentiellement pour les gommes de silicone, consiste à réaliser l'enrobage au sein d'un solvant dans lequel on a au préalable solubilisé la gomme de silicone et que l'on évapore ensuite pour réaliser l'enrobage.

La présente invention a également pour objet le procédé de préparation des microsphères chargées et enrobées.

Le chargement et l'enrobage peuvent être réalisés séparément ou simultanément selon divers procédés.

Le procédé le plus simple consiste à mélanger dans un récipient adapté sous agitation, les microsphères poreuses avec la substance de chargement hydrophile ou lipophile, puis d'ajouter après retour à l'état pulvérulent la substance d'enrobage à l'état liquide. Ce procédé s'applique essentiellement aux huiles perfluorées et aux huiles de silicones fluorées.

Lorsque la substance d'enrobage est dispersible dans la substance de chargement, comme c'est le cas pour les huiles perfluorées et les huiles de silicones fluorées dans les alcools polyhydroxylés tels que le glycérol ou les mélanges d'eau et d'un alcool polyhydroxylé comme le sorbitol, on peut réaliser le chargement et l'enrobage simultanément suivant le procédé précédent en mélangeant les microsphères et la dispersion.

Un autre procédé utilisé préférentiellement pour les gommes de silicone consiste à réaliser le chargement et l'enrobage au sein d'un solvant convenablement choisi.

Suivant ce procédé, le solvant doit en effet satisfaire à certaines conditions :
- sa température d'ébullition doit être la plus basse possible et inférieure à celle de la substance hydrophile, lipophile ou perfluorée,
- il ne doit pas former d'azéotrope important avec la substance de chargement,
- son affinité vis-à-vis des microsphères doit être négligeable par rapport à celle de la substance de chargement,
- enfin, il doit être peu ou pas toxique.

Parmi les solvants particulièrement appropriés pour la mise en oeuvre du procédé, on peut notamment citer l'éther diéthylique, le dichlorométhane et le n-hexane.

Suivant ce procédé, on introduit dans le solvant les microsphères, la substance de chargement hydrophile, lipophile ou perfluorée qui se retrouve à l'état de dispersion ou en solution suivant sa solubilité et on réalise le chargement par agitation manuelle ou mécanique ou en évaporant le solvant lorsque la substance de chargement est en solution.

On ajoute ensuite au système précédent, une solution ou une dispersion de la substance d'enrobage dans le solvant que l'on évapore afin d'enrober les microsphères chargées.

Il est à noter que l'étape de chargement peut être réalisée au sein du solvant en présence de la substance d'enrobage solubilisé ou en dispersion, à condition que l'affinité de la substance de chargement pour les microsphères soit supérieure à celle de la substance d'enrobage.

Il est particulièrement avantageux de réaliser l'étape de chargement au sein d'un solvant dans la mesure où ce procédé permet de réaliser des chargements impossibles ou très difficiles par mélange direct, d'obtenir des sphères chargées de façon plus homogène et surtout de favoriser la pénétration des substances de chargement à l'intérieur des pores, ce qui se traduit par un produit chargé poudreux, où les particules sont bien individualisées, et un taux optimum de charge des microsphères supérieur à celui obtenu par mélange direct.

Les microsphères enrobées, ou chargées et enrobées, se présentent sous la forme d'un produit ayant une consistance poudreuse que l'on disperse ensuite dans une phase continue non miscible vis-à-vis de la substance d'enrobage et de préférence également non miscible vis-à-vis de la substance de chargement. Cette phase continue peut être un véhicule cosmétique ou pharmaceutique qui peut être une solution aqueuse, du type lotion, un gel, une émulsion huile-dans-l'eau ou eau-dans-l'huile, une phase anhydre au sens des rouges à lèvres, ou des liants utilisés dans les poudres, les fards à paupières ou à joues.

Dans les compositions cosmétiques selon l'invention, la proportion en microsphères enrobées ou éventuellement chargées et enrobées peut varier dans de larges limites mais est généralement comprise entre 0,1 et 60 % en poids et de préférence de 4 à 20 % en poids dans le cas des gels et de 0,5 à 40 % en poids pour les compositions cosmétiques de maquillage.

On va maintenant donner à titre d'illustration plusieurs exemples de préparation des microsphères enrobées et des microsphères chargées et enrobées, ainsi que des exemples de compositions cosmétiques contenant de telles microsphères.

### PREPARATION DES MICROSPHERES

### I. Préparation de microsphères enrobées

### EXEMPLE 1 :

A 10 g de microsphères de poly-β-alanine réticulée, à l'état sec on introduit 10 g d'huile perfluorée vendue sous la dénomination de FOMBLIN HC25 par la Société MONTEFLUOS.

On agite l'ensemble à l'aide d'un agitateur Rayneri muni d'une défloculeuse jusqu'à l'obtention d'un mélange homogène.

On obtient ainsi un produit pulvérulent facilement dispersible dans une phase huileuse ou siliconée.

### II. Préparation de microsphères chargées et enrobées

### EXEMPLE 2 :

A 10 g de microsphères de poly-β-alanine réticulée, à l'état sec, on ajoute 20 g d'eau, de manière à obtenir 30 g de microsphères hydratées, puis on introduit 10 g d'huile perfluorée vendue sous la dénomination de FOMBLIN HC25 par la Société MONTEFLUOS.

On agite l'ensemble à l'aide d'un agitateur Rayneri muni d'une défloculeuse jusqu'à l'obtention d'un mélange homogène.

On obtient ainsi un produit pulvérulent facilement dispersible dans une phase huileuse ou siliconée.

### EXEMPLE 3 :

10 g de microsphères de poly-β-alanine réticulée sont chargés par 20 g de glycérol. On ajoute ensuite 10 g d'huile perfluorée vendue sous la dénomination de FOMBLIN HC25 par la Société MONTEFLUOS. On agite l'ensemble à l'aide d'un agitateur Rayneri muni d'une défloculeuse jusqu'à l'obtention d'un mélange homogène.

On obtient ainsi un produit pulvérulent facilement dispersible dans une phase huileuse ou siliconée.

### EXEMPLE 4 :

On mélange 2 g d'huile perfluorée vendue sous la dénomination de FOMBLIN Z TETRAOL par la Société MONTEFLUOS à 8 g d'huile perfluorée vendue sous la dénomination de FOMBLIN HC25 par la Société MONTEFLUOS.

Ce mélange est ensuite dispersé dans 20 g de glycérol, et l'ensemble est introduit avec 10 g de microsphères de poly-β-alanine réticulée, à l'état sec, dans un récipient adapté.

On agite l'ensemble à l'aide d'un agitateur Rayneri muni d'une défloculeuse en chauffant légèrement jusqu'à l'obtention d'un produit homogène, pulvérulent, facilement dispersible dans une phase huileuse ou siliconée.

### EXEMPLE 5 :

A 10 g de microsphères de poly-β-alanine réticulée, à l'état sec, on ajoute 30 g d'une dispersion constituée de 10 g d'huile perfluorée, vendue sous la dénomination de FOMBLIN Z 60 par la Société MONTEFLUOS, dans 20 g d'une solution constituée de 5 g de D-panthénol dans 15 g de glycérol. On agite l'ensemble à l'aide d'un agitateur Rayneri muni d'une défloculeuse jusqu'à l'obtention d'un produit homogène, pulvérulent, facilement dispersible dans une phase huileuse ou siliconnée.

### EXAMPLE 6 :

Dans un réacteur, on introduit 10 g de microsphères, vendues sous la dénomination de POLYTRAP Q5-6603 par la Société DOW CORNING que l'on chauffe à 70°C. Puis on ajoute 15 g d'acétate d'α-tocophérol préalablement chauffé sous azote à 70°C.

On agite l'ensemble sous azote, jusqu'à l'obtention d'un produit homogène, puis on ajoute 15 g d'huile perfluorée vendue sous la dénomination de FOMBLIN HC25 par la Société MONTEFLUOS.

Après homogénéisation, on obtient un produit pulvérulent facilement dispersible dans une phase hydrophile.

### EXEMPLE 7 :

A 10 g de microsphères de silicone vendues sous la dénomination de SILICONE POWDER X2-1605 par la Société DOW CORNING, on ajoute 10 g d'huile de silicone vendue sous la dénomination de DC FLUID 200 (0,1.10⁻⁴ m²/s) par la Société DOW CORNING.

On agite l'ensemble à l'aide d'un agitateur Rayneri muni d'une défloculeuse, jusqu'à l'obtention d'un produit pulvérulent, puis on ajoute 5 g d'huile perfluorée vendue sous la dénomination de FOMBLIN HC25 par la Société MONTEFLUOS.

On agite de nouveau dans les mêmes conditions que précédemment, et l'on obtient un produit poudreux facilement dispersible dans une phase hydrophile.

### EXEMPLE 8 :

A 10 g de microsphères de silice vendues sous la dénomination de SILCRON G640 par la Société CHIMILAB distributeur de la Société SCM, on ajoute 30 g d'une dispersion constituée de 10 g d'huile perfluorée vendue sous la dénomination de FOMBLIN HC25 par la Société MONTEFLUOS, dans 20 g de glycérol.

On agite l'ensemble à l'aide d'un agitateur Rayneri muni d'une défloculeuse, en chauffant légèrement jusqu'à l'obtention d'un produit pulvérulent facilement dispersible dans une phase huileuse ou siliconée.

### EXEMPLE 9 :

A 10 g de microsphères de poly-β-alanine réticulée, à l'état sec, on ajoute 30 g d'une dispersion constituée de 10 g d'huile de silicone fluorée, vendue sous la dénomination de FL100 (1.10⁻³ m²/s) par la Société SHINETSU, dans 20 g de glycérol.

On agite l'ensemble à l'aide d'un agitateur Rayneri muni d'une défloculeuse en chauffant légèrement jusqu'à l'obtention d'un mélange homogène. On obtient ainsi un produit pulvérulent facilement dispersible dans une phase huileuse.

### EXEMPLE 10:

Dans un réacteur, on introduit 10 g de microsphères, vendues sous la dénomination de POLYTRAP Q5-6603 par la Société DOW CORNING, que l'on chauffe à 70°C. Puis on ajoute 15 g d'acétate d'α-tocophérol préalablement chauffé sous azote à 70°C. On agite l'ensemble sous azote, jusqu'à l'obtention d'un produit homogène, puis on ajoute 15 g d'huile de silicone fluorée vendue sous la dénomination de FL100 (4,5.10⁻⁴ m²/s) par la Société SHINETSU.

Après homogénéisation, on obtient un produit pulvérulent facilement dispersible dans une phase hydrophile.

### EXEMPLE 11 :

On disperse dans 150 cm³ de n-hexane, 10 g de poly-β-alanine réticulée, à l'état sec, et 30 g d'un mélange (50/50) d'eau et de glycérol, puis on ajoute une solution de 5 g de gomme de silicone, vendue sous la dénomination de Sgm-3 par la Société DOW CORNING, dans 100 cm³ de n-hexane.

On agite l'ensemble jusqu'à disparition des gouttelettes de phase hydrophile, et on évapore alors le solvant sous vide et à température ambiante au rotavapor. On obtient après élimination totale du solvant, un produit qui redevient pulvérulent après un léger broyage, et qui est alors facilement dispersible dans une phase huileuse.

### EXEMPLE 12 :

A 10 g de microsphères de cellulose vendues par la Société CHISSO, on ajoute 10 g de glycérol. On agite l'ensemble à l'aide d'un agitateur Rayneri muni d'une défloculeuse en chauffant légèrement jusqu'à l'obtention d'un mélange homogène. Puis on disperse les microsphères ainsi gonflées dans 150 cm³ de n-hexane, et on ajoute une solution de 5 g de gomme de silicone, vendue sous la dénomination de Sgm-3 par la Société DOW CORNING, dans 100 cm³ de n-hexane. On évapore ensuite au rotavapor la totalité du solvant sous vide et à température ambiante de manière à réaliser l'enrobage.

On obtient alors un produit qui redevient pulvérulent après un léger broyage, et qui est alors facilement dispersible dans une phase huileuse.

### EXEMPLE 13 :

On disperse dans 150 cm³ de dichlorométhane, 10 g de microsphères, vendues sous la dénomination de POLYTRAP Q5-6603 par la Société DOW CORNING, et on ajoute une solution de 15 g d'acétate d'α-tocophérol et 5 g de gomme de silicone, vendue sous la dénomination de Sgm-3 par la Société DOW CORNING, dans 100 cm³ de dichlorométhane. On évapore ensuite au rotavapor la totalité du solvant sous vide et à température ambiante. On obtient alors un produit qui redevient pulvérulent après un léger broyage et qui est alors facilement dispersible dans une phase hydrophile.

### EXEMPLE 14 :

A 10 g de microsphères vendues sous la dénomination de POLYTRAP Q5-6603 par la Société DOW CORNING, on ajoute 20 g de perfluorotributylamine vendue sous la dénomination de FLUORINERT FC-43 par la Société 3M. On agite l'ensemble à l'aide d'un agitateur Rayneri muni d'une défloculeuse jusqu'à l'obtention d'un mélange homogène.

Puis on disperse les microsphères ainsi gonflées dans 150 cm² de n-hexane, et on ajoute une solution de 5 g de gomme de silicone, vendue sous la dénomination de SGM-3 par la Société DOW CORNING, dans 100 cm³ de n-hexane. On évapore ensuite au rotavapor la totalité du solvant sous vide et à température ambiante de manière à réaliser l'enrobage.

On obtient alors un produit qui redevient pulverulent après un léger broyage et qui est alors facilement dispersible dans une phase aqueuse ou huileuse.

### EXEMPLES DE COMPOSITIONS COSMETIQUES

### EXEMPLE 15 : Rouge à lèvres

| | |
|---|---|
| - Microsphères de Poly-β-alanine chargées et enrobées obtenues selon l'exemple 4 | 4 g |
| - Ozokérite | 14,4 g |
| - Cire microcristalline | 4,8 g |
| - Cire de Candellila | 7,2 g |
| - Huile de jojoba | 6,0 g |
| - Huile de ricin | 1,2 g |
| - Lanoline liquide | 18,0 g |
| - Lanoline acétylée | 9,6 g |
| - Huile de vaseline | 10,8 g |
| - Talc | 3,6 g |
| - Mica-titane | 8,4 g |
| - D et C Red 7 Ca lake | 5,04 g |
| - D et C Red 7 Ba lake | 2,76 g |
| - FDC Yellow 5 | 0,96 g |
| - Bioxyde de titane | 3,0 g |
| - Butylhydroxytoluène | 0,24 g |
| - Parfum q.s. | |

Les microsphères chargées et enrobées sont introduites en dernier dans la base de rouge à lèvres fondue à 90°C. La dispersion de ces microsphères est très rapide, permettant ainsi une incorporation et une dispersion très aisée des huiles fluorées d'enrobage, ce qui n'est pas le cas lorsque ces dernières sont introduites directement dans la base.

Après coulage et refroidissement, on obtient des sticks homogènes, onctueux, très doux et glissants à l'application, donnant un film très brillant sur les lèvres et ayant des propriétés hydratantes.

### EXEMPLE 16 : Rouge à lèvres

| | |
|---|---|
| - Microsphères de Poly-β-alanine chargées et enrobées obtenues selon l'exemple 11 | 4,5 g |
| - Ozokérite | 14,33 g |
| - Cire microcristalline | 4,78 g |
| - Cire de Candellila | 7,16 g |
| - Huile de jojoba | 5,97 g |
| - Huile de ricin | 1,19 g |
| - Lanoline liquide | 17,91 g |
| - Lanoline acétylée | 9,55 g |
| - Huile de vaseline | 10,75 g |
| - Talc | 3,58 g |
| - Mica titane | 8,36 g |
| - D et C Red 7 Ca lake | 5,01 g |
| - D et C Red 7 Ba lake | 2,74 g |
| - FDC Yellow 5 | 0,96 g |
| - Bioxyde de titane | 2,98 g |
| - Butylhydroxytoluène | 0,23 g |
| - Parfum q.s. | |

Les microsphères chargées et enrobées sont introduites en dernier dans la base de rouge à lèvres fondue à 80-85°C. La dispersion de ces microsphères est très rapide, permettant ainsi une incorporation et une dispersion très aisée de la gomme de silicone d'enrobage, ce qui est impossible lorsque cette dernière est introduite directement dans cette base qui ne contient pas d'huile de silicone.

Après coulage et refroidissement, on obtient des sticks homogènes, onctueux, très doux et glissants à l'application, donnant un film très brillant sur les lèvres, et ayant des propriétés hydratantes.

### EXEMPLE 17 : Fard à paupières

| | |
|---|---|
| - Microsphères de Poly-β-alanine chargées et enrobées obtenues selon l'exemple 3 | 4 g |
| - Pigments | 30,0 g |
| - Micatitane | 50,0 g |
| - Lanoline liquide | 5,0 g |
| - Huile de vaseline | 4,8 g |
| - Parahydroxybenzoate de propyle | 0,2 g |
| - Talc q.s.p. | 100,0 g |

Le fard à paupières obtenu est doux et a un bon pouvoir couvrant.

### EXEMPLE 18 : Gel

| | |
|---|---|
| - Microsphères de Polytrap Q5-6603 chargées et enrobées obtenues selon l'exemple 6 | 3,5 g |
| - Carbopol 941 | 0,50 g |
| - Triéthanolamine q.s.p. pH = 6 | |
| - Eau q.s.p. | 100,0 g |

Les microsphères chargées et enrobées, sont dispersées avant la neutralisation par la triéthanolamine, lorsque la viscosité du système est encore faible, ce qui permet de conserver lors de la gélification, un bon état de dispersion des microsphères et en conséquence de l'huile perfluorée d'enrobage, ce qui est difficile à réaliser par introduction directe de l'huile perfluorée dans le gel.

### EXEMPLE 19 : Composition du type "oil-free"

| | | |
|---|---|---|
| (A) | - Microsphères de Polytrap Q5-6603 chargées et enrobées obtenues selon l'exemple 6 | 4 g |
| | - Carbopol 940 | 0,58 g |
| (B) | - Carbopol EX 183 | 0,10 g |
| | - Eau | 60,0 g |
| | - Glycérol | 1,92 g |
| (C) | - Conservateur | 0,19 g |
| | - Eau | 17,08 g |
| (D) | - Triéthanolamine | 0,77 g |
| (E) | - Cyclopentadimethylsiloxane | 15,36 g |

Les microsphères chargées et enrobées (A) sont dispersées dans (B) à température ambiante, puis on ajoute la phase (C) préalablement chauffée à 80°C et on neutralise à la triéthanolamine (D), la silicone volatile (E) étant dispersée en dernier à température ambiante.

On obtient ainsi une composition dont la dispersion des microsphères chargées et enrobées est parfaitement homogène, et la présence du perfluoropolyméthylisopropyl éther (FOMBLIN HC25) sous forme d'enrobage des microsphères chargées, donne à la composition un toucher très agréable, en particulier au niveau de la douceur, et de bonnes propriétés d'étalement.

### EXEMPLE 20 : Fard à joues

| | |
|---|---|
| - Microsphères de Poly-β-alanine chargées et enrobées obtenues selon l'exemple 4 | 4 g |
| - Huile de paraffine | 54,25 g |
| - Huile de vaseline | 11,20 g |
| - Cire de carnauba | 5,60 g |
| - Cire de polyéthylène | 5,60 g |
| - Micatitane | 11,20 g |
| - Dioxyde de titane | 5,60 g |
| - D et C Red n° 7 | 0,20 g |
| - Oxyde de fer | 2,20 g |
| - Parahydroxybenzoate de propyle | 0,10 g |
| - Butylhydroxytoluène | 0,05 g |

## Revendications

1. Microsphères enrobées, caractérisées par le fait qu'elles sont constituées de microsphères poreuses comportant une substance d'enrobage fixée à la surface externe desdites microsphères, ladite substance étant choisie parmi une huile perfluorée, une huile de silicone fluorée et une gomme de silicone ayant la formule générale suivante : 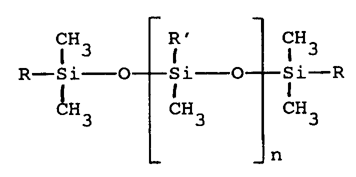 dans laquelle :
R représente -CH₃, -OH ou -CH=CH₂, et
R' représente -CH₃ ou -C₆H₅
n étant tel que la viscosité est > 1.10⁻² m²/s à 25°C.

2. Microsphères selon la revendication 1, caractérisées par le fait que les microsphères poreuses présentent des tailles moyennes de particules inférieures à 800 »m et de préférence comprises entre 0,1 et 300 »m.

3. Microsphères selon la revendication 1 ou 2, caractérisées par le fait que les microsphères poreuses sont choisies parmi le poly-β-alanine, les polyacrylates, les polyamides, les polyméthacrylates, les polyéthylènes, les polypropylènes, les copolymères styrène-divinylbenzène, les celluloses, les silicones , les silices et les poudres d'hydroxyapatite.

4. Microsphères selon l'une quelconque des revendications précédentes, caractérisées par le fait que l'enrobage des microsphères poreuses est réalisé à l'aide de 0,1 à 500 % en poids d'huile perfluorée, d'huile de silicone fluorée ou de gomme de silicone par rapport au poids des microsphères poreuses.

5. Microsphères selon l'une quelconque des revendications précédentes, caractérisées par le fait que l'huile perfluorée est un perfluoroalcane de formule :
CₙF₂ₙ₊₂
avec n > 19

6. Microsphères selon l'une quelconque des revendications 1 à 4, caractérisées par le fait que l'huile perfluorée est un perfluoropolyéther répondant à l'une des formules suivantes :
(a) 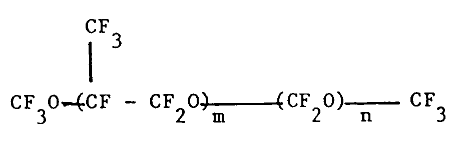 avec le rapport m/n : 5 à 40
(b) RCF₂O-(C₂F₄O)ₚ-(CF₂O)_{q}-CF₂R
avec le rapport p/q = 0,5 à 1,5
R représente un atome de fluor, -COOH, -COOCH₃, -CH₂OH, ou -CH₂O-CH₂-CHOH-CH₂OH et -CH₂(OCH₂CH₂)ₚ-OH avec p = 1 ou 2.
(c) 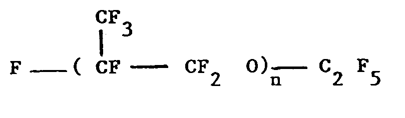 avec n = 10 - 500
(d) 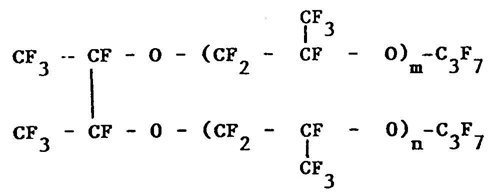 dans laquelle :
m et n sont des nombres entiers de 0 à 3, tels que le poids moléculaire est supérieur à 1000.

7. Microsphères selon l'une quelconque des revendications 1 à 4, caractérisées par le fait que l'huile de silicone fluorée est choisie parmi l'une des formules suivantes :
(a) 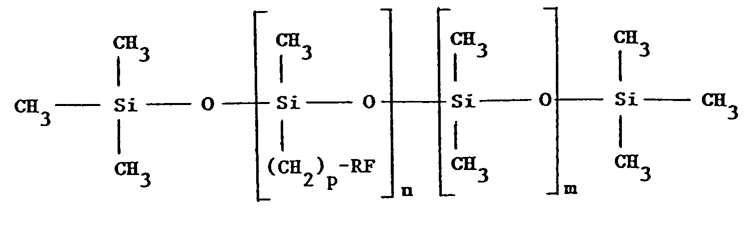 dans laquelle :
n est un nombre entier de 1 à 300,
m est un nombre entier de 0 à 150,
p est un nombre entier de 0 à 5 et
RF est un radical perfluoroalkyle ayant de 1 à 8 atomes de carbone.
(b) 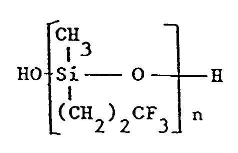 avec n > 3

8. Microsphères selon l'une quelconque des revendications précédentes, caractérisées par le fait que les pores des microsphères poreuses sont chargées à l'aide d'une phase hydrophile, lipophile ou perfluorée préalablement à leur enrobage.

9. Microsphères selon la revendication 8, caractérisées par le fait que la phase hydrophile est de l'eau, un alcool polyhydroxylé ou un mélange d'eau et d'un alcool polyhydroxylé.

10. Microsphères selon la revendication 9, caractérisées par le fait que l'alcool polyhydroxylé est choisi parmi le glycérol et le propanediol-1,2.

11. Microsphères selon l'une quelconque des revendications 8 à 10, caractérisées par le fait que la phase hydrophile contient au moins un actif hydrosoluble choisi parmi le D-panthénol, le polyméthacrylate de glycéryle, le hyaluronate de sodium, le sel de sodium de l'acide pyroglutamique et les vitamines.

12. Microsphères selon la revendication 8, caractérisées par le fait que la phase lipophile est de l'huile de paraffine, de l'huile de jojoba, de l'huile de ricin, du squalane, le myristate d'isopropyle ou une huile de silicone.

13. Microsphères selon la revendication 12, caractérisées par le fait que la phase lipophile contient au moins un actif liposoluble choisi parmi l'acétate d'α-tocophérol, le nicotinate de tocophérol, le β-carotène, le N,N-diéthyltoluamide, les filtres U.V. liposolubles, le ricinoléate de zinc, les antioxydants, les conservateurs.

14. Microsphères selon la revendication 8, caractérisées par le fait que la phase perfluorée est constituée d'un perfluoroalcane ou d'un perfluoropolyéther de poids moléculaire inférieur à 1.000.

15. Composition cosmétique caractérisée par le fait qu'elle contient dans un véhicule cosmétique approprié des microsphères enrobées selon l'une quelconque des revendications 1 à 14.

16. Composition cosmétique selon la revendication 15, caractérisée par le fait que la proportion de microsphères est comprise entre 0,1 et 60 % en poids.

## Claims

1. Coated microspheres, characterized in that they consist of porous microspheres containing a coating substance attached to the external surface of the said microspheres, the said substance being chosen from a perfluorinated oil, a fluorinated silicone oil and a silicone gum having the following general formula: 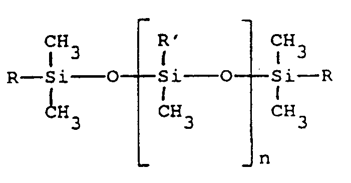 in which:
R represents -CH₃, -OH or -CH=CH₂, and
R' represents -CH₃ or -C₆H₅
n being such that the viscosity is > 1 × 10⁻² m²/s at 25°C.

2. Microspheres according to Claim 1, characterized in that the porous microspheres have mean particle sizes of less than 800 »m and preferably of between 0.1 and 300 »m.

3. Microspheres according to Claim 1 or 2, characterized in that the porous microspheres are chosen from poly-β-alanine, polyacrylates, polyamides, polymethacrylates, polyethylenes, polypropylenes, styrenedivinylbenzene copolymers, celluloses, silicones, silicas and hydroxyapatite powders.

4. Microspheres according to any one of the preceding claims, characterized in that the coating of the porous microspheres is performed with the aid of 0.1 to 500% by weight of perfluorinated oil, of fluorinated silicone oil or of silicone gum relative to the weight of the porous microspheres.

5. Microspheres according to any one of the preceding claims, characterized in that the perfluorinated oil is a perfluoroalkane of formula:
CₙF₂ₙ₊₂
with n > 19

6. Microspheres according to any one of Claims 1 to 4, characterized in that the perfluorinated oil is a perfluoropolyether corresponding to one of the following formulae:
(a) 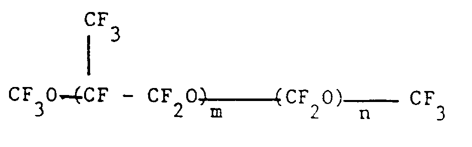 with the m/n ratio: 5 to 40
(b) RCF₂O-(C₂F₄O)ₚ-(CF₂O)_{q}-CF₂R
with the p/q ratio = 0.5 to 1.5
R represents a fluorine atom, -COOH, -COOCH₃, -CH₂OH, or -CH₂O-CH₂-CHOH-CH₂OH and -CH₂(OCH₂CH₂)ₚ-OH with p = 1 or 2.
(c) 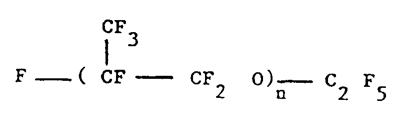 with n = 10 - 500
(d) 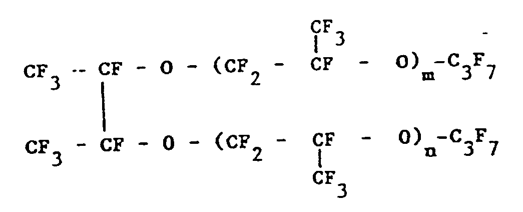 in which:
m and n are integers from 0 to 3, such that the molecular weight is greater than 1000.

7. Microspheres according to any one of Claims 1 to 4, characterized in that the fluorinated silicone oil is chosen from one of the following formulae:
(a) 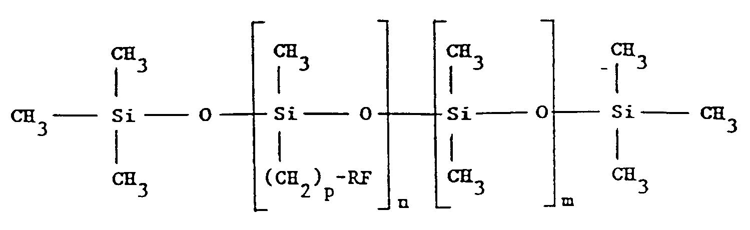 in which:
n is an integer from 1 to 300,
m is an integer from 0 to 150,
p is an integer from 0 to 5 and
RF is a perfluoroalkyl radical having from 1 to 8 carbon atoms.
(b) 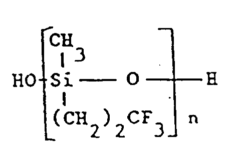 with n > 3

8. Microspheres according to any one of the preceding claims, characterized in that the pores of the porous microspheres are charged with the aid of a hydrophilic, lipophilic or perfluorinated phase prior to their coating.

9. Microspheres according to Claim 8, characterized in that the hydrophilic phase is water, a polyhydroxylated alcohol or a mixture of water and a polyhydroxylated alocohol.

10. Microspheres according to Claim 9, characterized in that the polyhydroxylated alcohol is chosen from glycerol and 1,2-propanediol.

11. Microspheres according to any one of Claims 8 to 10, characterized in that the hydrophilic phase contains at least one water-soluble active agent chosen from D-panthenol, polyglyceryl methacrylate, sodium hyalurona te, the sodium salt of pyroglutamic acid and vitamins.

12. Microspheres according to Claim 8, characterized in that the lipophilic phase is paraffin oil, jojoba oil, castor oil, squalane, isopropyl myristate or silicone oil.

13. Microspheres according to Claim 12, characterized in that the lipophilic phase contains at least one fat-soluble active agent chosen from α-tocopherol acetate, tocopherol nicotinate, β-carotene, N,N-diethyltoluamide, fat-soluble UV screening agents, zinc ricinoleate, antioxidants and preservatives.

14. Microspheres according to Claim 8, characterized in that the perfluorinated phase consists of a perfluoroalkane or a perfluoropolyether having a molecular weight of less than 1,000.

15. Cosmetic composition characterized in that it contains, in an appropriate cosmetic vehicle, coated microspheres according to any one of Claims 1 to 14.

16. Cosmetic composition according to Claim 15, characterized in that the proportion of microspheres is between 0.1 and 60% by weight.

## Patentansprüche

1. Überzogene Mikrokügelchen, dadurch gekennzeichnet, daß sie aus porösen Mikrokügelchen bestehen, die mit einer Umhüllungssubstanz überzogen sind, die auf der äußeren Oberfläche der Mikrokügelchen fixiert ist, wobei diese Substanz aus einem perfluorierten Öl, einem fluorierten Silikonöl sowie einem Silikongummi der folgenden allgemeinen Formel ausgewählt ist: 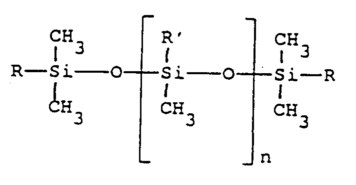 worin
R die Gruppe -CH₃, -OH oder -CH=CH₂ und
R' die Gruppe -CH₃ oder -C₆H₅ bedeuten und
n einen solchen Wert aufweist, daß die Viskosität 1 · 10² m²/s bei 25 °C beträgt.

2. Mikrokügelchen nach Anspruch 1, dadurch gekennzeichnet, daß die porösen Mikrokügelchen einen mittleren Teilchendurchmesser von weniger als 800 »m, vorzugsweise 0,1 bis 300 »m aufweisen.

3. Mikrokügelchen nach Anspruch 1 oder 2, dadurch gekennzeichnet, daß die porösen Mikrokügelchen unter Poly-β-alanin, Polyacrylaten, Polyamiden, Polymethacrylaten, Polyethylenen, Polypropylenen, Styrol/Divinylbenzol-Copolymeren, Cellulosen, Silikonen, Kieselerden und Hydroxyapatitpulvern ausgewählt sind.

4. Mikrokügelchen nach einem der vorstehenden Ansprüche, dadurch gekennzeichnet, daß das Überziehen der porösen Mikrokügelchen mit Hilfe von 0,1 bis 500 Gew.% perfluoriertem Öl, fluoriertem Silikonöl oder Silikongummi in bezug auf das Gewicht der porösen Mikrokügelchen erfolgt.

5. Mikrokügelchen nach einem der vorstehenden Ansprüche, dadurch gekennzeichnet, daß das perfluorierte Öl ein Perfluoroalkan der Formel
CₙF₂ₙ₊₂
darstellt, wobei n > 19.

6. Mikrokügelchen nach einem der Ansprüche 1 bis 4, dadurch gekennzeichnet, daß das perfluorierte Öl einen Perfluorpolyether gemäß einer der folgenden Formeln darstellt:
(a) 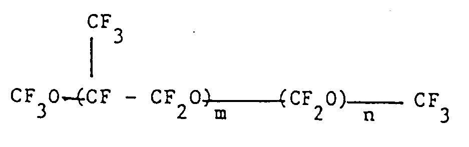 wobei das Verhältnis m/n 5 bis 40 beträgt,
b) RCF₂O-(C₂F₄O)ₚ-(CF₂O)_{q}-CF₂R
wobei das Verhältnis p/q 0,5 bis 1,5 beträgt,
R ein Fluoratom, -COOH, -COOCH₃, -CH₂OH oder -CH₂O-CH₂-CHOH-CH₂OH und -CH₂(OCH₂CH₂)ₚ-OH bedeutet, wobei
p 1 oder 2 ist;
(c) 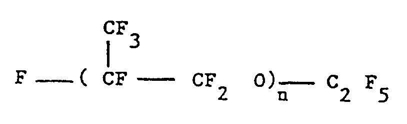 wobei n = 10 - 500 beträgt;
(d) 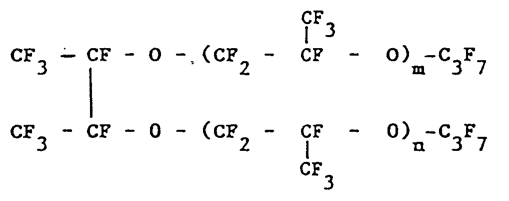 worin:
m und n ganze Zahlen zwischen 0 und 3 bedeuten, wobei das Molekulargewicht mehr als 1000 beträgt.

7. Mikrokügelchen nach einem der Ansprüche 1 bis 4, dadurch gekennzeichnet, daß das fluorierte Silikonöl aus einer der Verbindungen mit den jeweiligen allgemeinen Formeln ausgewählt ist:
(a) 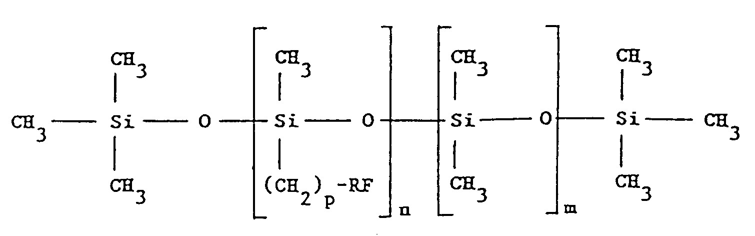 worin:
n eine ganze Zahl von 1 bis 300,
m eine ganze Zahl von 0 bis 150,
p eine ganze Zahl von 0 bis 5 bedeuten und
RF einen Perfluoralkylrest mit 1 bis 8 Kohlenstoffatomen darstellt;
(b) 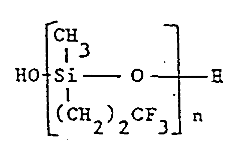 wobei n > 3.

8. Mikrokügelchen nach einem der vorstehenden Ansprüche, dadurch gekennzeichnet, daß die Poren der porösen Mikrokügelchen vor dem Überzugsprozeß mit einer hydrophilen, lipophilen oder perfluorierten Phase beladen worden sind.

9. Mikrokügelchen nach Anspruch 8, dadurch gekennzeichnet, daß die hydrophile Phase Wasser, ein polyhydroxylierter Alkohol oder ein Gemisch aus Wasser und einem polyhydroxyliertem Alkohol ist.

10. Mikrokügelchen nach Anspruch 9, dadurch gekennzeichnet, daß der polyhydroxylierte Alkohol aus Glycerin und 1,2-Propandiol ausgewählt ist.

11. Mikrokügelchen nach einem der vorstehenden Ansprüche 8 bis 10, dadurch gekennzeichnet, daß die hydrophile Phase mindestens einen wasserlöslichen Wirkstoff enthält, der aus D-Panthenol, Glycerinpolymethacrylat, Natriumhyaluronat,dem Natriumsalz der Pyroglutaminsäure und Vitaminen ausgewählt ist.

12. Mikrokügelchen nach Anspruch 8, dadurch gekennzeichnet, daß die lipophile Phase ein Paraffinöl, Jojobaöl, Rizinusöl, Squalan, Isopropylmyristat oder ein Silikonöl darstellt.

13. Mikrokügelchen nach Anspruch 12, dadurch gekennzeichnet, daß die lipophile Phase mindestens einen fettlöslichen Wirkstoff enthält, der aus d'-α-Tocopherol, Tocopherolnikotinat, β-Karotin, N,N-Diethyltoluamid, fettlöslichen UV-Filtern, Zinkricinoleat, Antioxidantien oder Konservierungsmitteln ausgewählt ist.

14. Mikrokügelchen nach Anspruch 8, dadurch gekennzeichnet, daß die perfluorierte Phase aus einem Perfluoralkan oder einem Perfluorpolyether mit einem Molekulargewicht von weniger als 1000 besteht.

15. Kosmetische Zubereitung, dadurch gekennzeichnet, daß sie in einem geeigneten kosmetischen Träger überzogene Mikrokügelchen nach einem der Ansprüche 1 bis 14 enthält.

16. Kosmetische Zubereitung nach Anspruch 15, dadurch gekennzeichnet, daß dem Anteil an Mikrokügelchen zwischen 0,1 und 60 Gew.% beträgt.
